**Europäisches Patentamt**

**European Patent Office**

**Office européen des brevets**

(19)

(11) Veröffentlichungsnummer : **0 114 025
B1**

(12) **EUROPÄISCHE PATENTSCHRIFT**

(45) Veröffentlichungstag der Patentschrift :
**25.03.87**

(21) Anmeldenummer : **83730122.5**

(22) Anmeldetag : **13.12.83**

(51) Int. Cl.⁴ : **A 61 F   2/24**, B 29 C 41/14

(54) **Verfahren zur Herstellung künstlicher Herzklappen.**

(30) Priorität : **27.12.82 DE 3248560**

(43) Veröffentlichungstag der Anmeldung :
**25.07.84 Patentblatt 84/30**

(45) Bekanntmachung des Hinweises auf die Patenterteilung : **25.03.87 Patentblatt 87/13**

(84) Benannte Vertragsstaaten :
**AT BE CH FR GB IT LI LU NL SE**

(56) Entgegenhaltungen :
**FR-A- 2 256 730
FR-A- 2 403 788
US-A- 2 017 604
BIOMEDIZINISCHE TECHNIK, Band 23, Nr. 7/8,
Juli/August 1978, Seiten 173-176 M. WASCHMANN:
"Ein neues Verfahren zur Herstellung von integrierten Taschenklappen für künstliche Blutpumpen"**

(73) Patentinhaber : **Hennig, Ewald, Dr.
Hirschhornerweg 17
D-1000 Berlin 37 (DE)**

(72) Erfinder : **Hennig, Ewald, Dr. med.
Hirschhornerweg 17
D-1000 Berlin 37 (DE)**
Erfinder : **Bücherl, Emil Sebastian Prof. Dr. Med.
Wangenheimstrasse 26
D-1000 Berlin 33 (DE)**
Erfinder : **Zartnack, Friedrich, Dipl.-Ing.
Grolmannstrasse 21
D-1000 Berlin 12 (DE)**

(74) Vertreter : **Meissner, Peter E., Dipl.-Ing. et al
Herbertstrasse 22
D-1000 Berlin 33 (DE)**

EP 0 114 025 B1

**Beschreibung**

Die Erfindung betrifft ein Verfahren zum Herstellen einer künstlichen Herzklappe, bestehend aus einem vorgefertigten Klappengehäuse und einem oder mehreren im Inneren des Gehäuses angeordneten Klappensegel(n), bei dem das oder die Klappensegel durch ein- oder mehrmaliges Eintauchen eines mit entsprechend der Form der Klappensegel geformten Flächen versehenen Kernes in eine Polymerlösung auf dem Kern gebildet werden.

Aus « Biomedizinische Technik » (Band 23, Heft 7-8, 1978, Seiten 173-176) sind verschiedene Verfahren zur Herstellung von Klappensegeln bekannt. So können die Klappensegel, die durch ein- oder mehrmaliges Eintauchen eines entsprechend geformten Kernes in z. B. eine Polyurethanlösung hergestellt worden sind, mit dem Klappengehäuse verklebt werden. Es ist hieraus auch bekannt, mittels eines zweiteiligen Kernes erst die Klappensegel durch Tauchen auszubilden und anschließend — nach Einsetzen des anderen Kernteiles — eine Art Klappengehäuse herzustellen, und zwar ebenfalls durch Tauchen, wobei sich bei diesem Vorgang die Übergänge der Klappensegel mit dem Klappengehäuse verbinden. Beim ersten Verfahren, also dem Ankleben, ergeben sich zwangsläufig an den Übergängen zwischen Klappensegel und Klappengehäuse Klebstoffrückstände und damit Unebenheiten an den Oberflächen. Das inhomogene Material (Klebstoff) und die unebenen Oberflächen führen beim Einsatz dieser Herzklappen dazu, daß innerhalb von kürzester Zeit Ablagerungen von zellulären Blutbestandteilen mit nachfolgender Kalzifizierung auftreten, die den Blutdurchgang vermindern, bzw. schließlich verhindern. Das zweite Verfahren ist relativ aufwendig, weil sehr genau abgestimmte Teilkerne verwendet werden müssen. Außerdem können Schichtdickenunterschiede auftreten, die dann zu unregelmäßigen Beanspruchungen führen.

Aus der FR-A-24 03 788 ist ein Verfahren der eingangs genannten Art zur Herstellung von künstlichen Herzklappen bekannt, wobei diese bestimmte Abmessungsparameter einhalten sollen. Die Herstellung kann im Tauchverfahren erfolgen. Auch hierbei wird darauf hingewiesen, daß die Anzahl der Tauchvorgänge die Membrandicke bestimmt.

Es ist daher die Aufgabe der Erfindung, künstliche Herzklappen herzustellen, die hinsichtlich des verwendeten Materials homogen sind, und bei denen an den Übergangsbereichen der Klappensegel zum Klappengehäuse keine Ablagerungen fördernde Unebenheiten vorhanden sind.

Gelöst wird diese Aufgabe erfindungsgemäß durch die im Patentanspruch 1 angegebenen Verfahrensschritte.

Ausgestaltungen ergeben sich aus den abhängigen Ansprüchen 2 und 3.

Der besondere Vorteil des erfindungsgemäßen Verfahrens besteht darin, daß einerseits das Klappengehäuse und die Klappensegel aus dem gleichen Material bestehen können und andererseits aber auch durch die Ausbildung eines die Klappensegel mit dem Gehäuse verbindenden Überzuges beim zweiten Tauchvorgang (Schritte c und d) Inhomogenitäten vermieden werden und auch keine Unebenheiten in der Oberfläche auftreten, die beim späteren Einsatz der Herzklappen Anlagerungsbereiche für Kalke und Phosphate ergeben würden. Darüberhinaus ermöglicht das Tauchverfahren die Herstellung sehr dünner, filmartiger Überzüge und damit die Herstellung von Klappensegeln sehr geringer Wandstärke.

Denkbar ist natürlich auch, daß die Klappensegel und das Gehäuse an sich aus verschiedenen Materialien bestehen, die dann durch einen homogenen Überzug — und damit ohne die beim Stand der Technik vorhandenen Nachteile — verbunden werden.

Das erfindungsgemäße Verfahren soll nachfolgend anhand der Zeichnungen erläutert werden. Dabei zeigt :

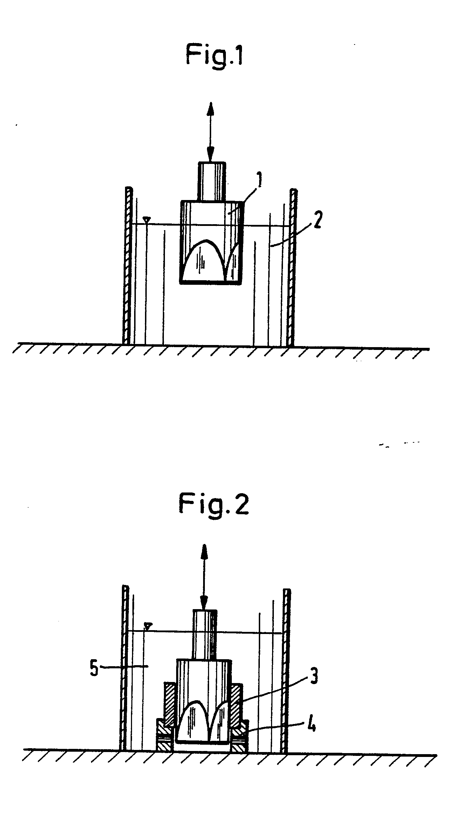

Figur 1 die Herstellung der Klappensegel durch Tauchen in eine erste Polymerlösung,

Figur 2 die Verbindung der Klappensegel mit dem Klappengehäuse in einer zweiten Polymerlösung,

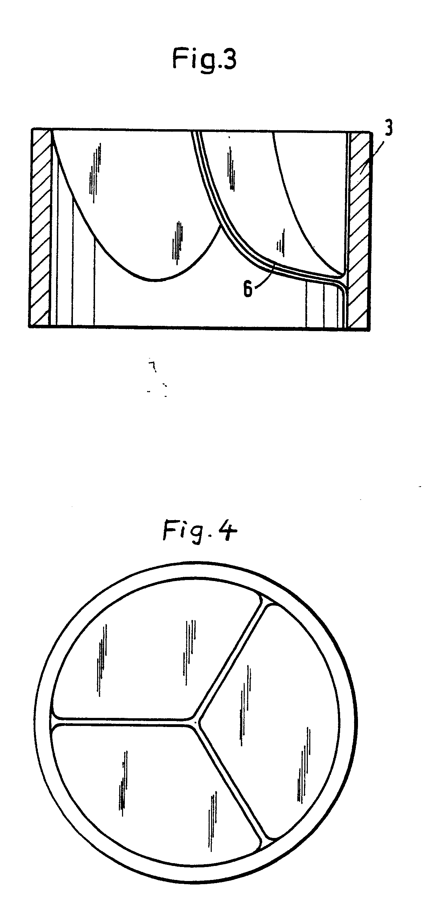

Figur 3 eine schematische Skizze durch einen Teilquerschnitt eines Klappenringes mit einem Klappensegel und

Figur 4 einen Grundriß von Figur 3.

Der für das erfindungsgemäße Verfahren verwendete Kern 1 kann beispielsweise aus Edelstahl oder aus Kunststoff bestehen. Dieser Kern weist entsprechend den zu bildenden Klappensegeln Formflächen auf, die im Falle eines Kernes aus Stahl hochglanzpoliert sind, bzw. bei einem Kern aus Kunststoff ggf. mit einem Überzug versehen sein können, der eine hochglatte Oberfläche für die erzeugende bzw. erzeugenden Polymerschicht(en) bildet. Im dargestellten Fall handelt es sich um die Herstellung einer sogenannten trileaflet Herzklappe, und daher weist der Kern drei entsprechende Flächen für die Klappensegel auf. Dieser Kern wird in eine erste Polymerlösung 2 mit einer Viskosität im Bereich von 24-192 Pa · s abgesenkt and zwar mit einer sehr geringen Absenkgeschwindigkeit, die verhindert, daß dabei Blasen oder dergleichen entstehen und Inhomogenitäten in dem sich auf dem Kern bildenden Polymer. Nach vollständigem Tauchen wird der Kern 1 mit dem darauf befindlichen Film aus der Lösung 2 vorsichtig herausgezogen und anschließend getrocknet. Dieser Vorgang kann ein oder mehrmals wiederholt werden, je nachdem welche Anzahl von Schichten erreicht werden soll bzw. welche Endwandstärke gewünscht wird.

In einer zweiten Polymerlösung 5 mit niedrigerer Viskosität im Bereich von 1,5-2 Pa · s befindet sich das vorgefertigte Klappengehäuse 3 (wobei dieses im Prinzip ebenfalls durch Tauchen eines entsprechenden Kernes in eine Polymerlösung hergestellt worden sein kann), und zwar wird dieses Klappengehäuse 3 so in der Lösung gehalten, daß sich unterhalb Ausströmöffnungen 4 befinden, durch die die Lösung aus dem Klappengehäuseinneren ausströmen kann. Der zwischenzeitlich getrocknete mit einer oder mit mehreren Schichten überzogene Kern 1 wird dann wiederum langsam in die zweite Polymerlösung 5 eingetaucht und in den in dieser Lösung gehaltenen Klappenring 3 eingeführt. Wie vorher angegeben, kann bei diesem Einführen die aus dem Inneren des Klappengehäuses verdrängte Flüssigkeit ausströmen, so daß auf diese Weise gewährleistet ist, daß keinerlei Blasen oder sonstige Inhomogenitäten zwischen Kern 1 und Klappengehäuse 3 verbleiben. Der Innendurchmesser des Klappengehäuses ist nur geringfügig größer als der Außendurchmesser des Kernes. Nach dem Einführen des Kernes in das Klappengehäuse verbleiben beide kurzzeitig in der Lösung.

Anschließend wird der Kern mit dem Klappengehäuse aus der Lösung entfernt und wiederum getrocknet. Nach dieser Trocknung läßt sich der Kern leicht von der so hergestellten Herzklappe entfernen, und diese kann ggf. noch nachbearbeitet werden, um beispielsweise verfahrensbedingte Ränder am Klappengehäuse zu entfernen. Es sei abschließend noch darauf hingewiesen, daß es für die Erfindung vorteilhaft ist, daß die Flächen am Kern, die zur Bildung der Klappensegel dienen, bzw. die axiale Erstreckung des Kernes in diesem Bereich etwas größer ist als die axiale Länge des Klappengehäuses.

**Patentansprüche**

1. Verfahren zum Herstellen einer künstlichen Herzklappe, bestehend aus einem vorgefertigten Klappengehäuse (3) und einem oder mehreren im Inneren des Gehäuses angeordneten Klappensegel(n) (6), bei dem das oder die Klappensegel durch ein- oder mehrmaliges Eintauchen eines mit entsprechend der Form der Klappensegel geformten Flächen versehenen Kernes (1) in eine Polymerlösung (2) auf dem Kern gebildet werden, dadurch gekennzeichnet, daß

a) der Kern (1) zur Herstellung der Klappensegel (6) langsam in eine erste Polymerlösung (2) mit einer Viskosität von 24-192 Pa · s eingetaucht und nach der Ausbildung mindestens einer Polymerschicht auf dem Kern (1) langsam aus der Lösung (2) herausgezogen wird, daß

b) der Kern (1) mit der darauf befindlichen Polymerschicht bzw. -schichten getrocknet wird, daß

c) in eine zweite Polymerlösung (5) mit einer Viskosität von 1,5-2 Pa · s das vorgefertigte Klappengehäuse (3), dessen Innendurchmesser geringfügig größer als der Außendurchmesser des Kernes ist, eingetaucht und in dieser Polymerlösung gehalten wird, daß

d) der Kern (1) mit der oder den Polymerschichten langsam in die zweite Polymerlösung (5) und das in der Lösung gehaltene Klappengehäuse (3) eingeführt wird, wobei die beim Einführen des Kernes (1) in den Innenraum des Klappengehäuses (3) verdrängte Polymerlösung aus dem Innenraum kontinuierlich ausströmt und sich dann auf den Polymerschichten am Kern (1) und dem Klappengehäuse (3) ein diese verbindender Überzug bildet, und daß

e) die Herzklappe — nach dem langsamen Herausziehen des Kernes (1) und des damit verbundenen Klappengehäuses (3) aus der zweiten Polymerlösung (5) und nach der Trocknung des Überzugs — vom Kern (1) abgezogen wird.

2. Verfahren nach Anspruch 1, dadurch gekennzeichnet, daß der Kern (1) aus Stahl mit hochglanzpolierten Flächen oder aus einem mit einem hochglatte Oberflächen bildenden Überzug versehenem Kunststoff besteht.

3. Verfahren nach Anspruch 1, dadurch gekennzeichnet, daß als Lösung eine Polyurethanlösung verwendet wird.

**Claims**

1. Method for the production of an artificial cardiac valve, consisting of a prefabricated valve housing (3) and one or more valve flaps (6) arranged in the interior of the housing, in which by the immersion once or several times of a core (1), which is provided with surfaces formed corresponding to the shape of the valve flaps, into a polymer solution (2), the valve flap or flaps are formed on the core, characterized in that

a) the core (1) for the production of the valve flaps (6) is slowly immersed into a first polymer solution (2) with a viscosity of 24-192 Pa · s and after the formation of at least one polymer layer on the core (1), it is slowly drawn out from the solution (2), that

b) the core (1) with the polymer layer or respectively layers situated thereon is dried, that

c) the prefabricated valve housing (3), the internal diameter of which is slightly greater than the external diameter of the core, is immersed into a second polymer solution (5) with a viscosity of 1.5-2 Pa · s and is held in this polymer solution, that

d) the core (1) with the polymer layer or layers is slowly introduced into the second polymer solution (5) and the valve housing (3) which is held in the solution, whereby the polymer solution which is displaced on introduction of the core (1) into the interior of the valve housing (3) continuously flows out of the interior and then on the polymer layers on the core (1) and the valve housing (3) forms a coating connecting them, and that

e) after the slow drawing out of the core (1) and of the valve housing (3) connected therewith from the second polymer solution (5) and after the drying of the coating, the cardiac valve is removed from the core (1).

2. Method according to Claim 1, characterized in that the core (1) consists of steel with mirror-finished surfaces or of a plastic which is provided with a coating forming highly smooth surfaces.

3. Method according to Claim 1, characterized in that a polyurethane solution is used as solution.

**Revendications**

1. Procédé pour la réalisation d'une valve cardiaque artificielle, constituée d'un siège de valve (3) préfabriqué et d'une ou plusieurs membranes de valve (6) disposées à l'intérieur du siège, dans lequel, par l'intermédiaire d'un trempage unique ou multiple, dans une solution de polymère (2), d'un noyau (1) muni de surfaces conformées de façon correspondant à la forme des membranes de valve, sont formées les membranes de valve, caractérisé en ce que :

a) le noyau (1) pour la réalisation de la membrane de valve (6) est plongé lentement dans une première solution de polymère (2) ayant une viscosité de 24-192 Pa · s et, après la formation d'au moins une couche de polymère sur le noyau (1), ce dernier est retiré lentement de la solution (2),

b) le noyau (1) avec la ou les couches de polymère est séché,

c) le siège de valve préfabriqué (3), dont le diamètre interne est légèrement plus grand que le diamètre externe du noyau, est plongé dans une seconde solution de polymère (5) ayant une viscosité de 1,5-2 Pa · s, et est maintenu dans cette solution,

d) le noyau (1) avec la ou les couches de polymère est introduit lentement dans la seconde solution de polymère (5) et le siège de valve (3) maintenu dans la solution, la solution de polymère déplacée par introduction du noyau (1) à l'intérieur du siège de valve (3) s'écoulant continuellement de l'intérieur de celui-ci, et, alors, sur les couches de polymère sur le noyau (1) et sur le siège de valve (3), il se forme un revêtement reliant ceux-ci, et

e) la valve cardiaque est retirée du noyau (1), après retrait lent du noyau (1) et du siège de valve (3) qui y est lié de la seconde solution de polymère (5) et séchage du revêtement.

2. Procédé selon la revendication 1, caractérisé en ce que le noyau (1) est constitué d'acier ayant des surfaces avec un poli miroir, ou d'une matière synthétique munie d'un revêtement formant une surface très lisse.

3. Procédé selon la revendication 1, caractérisé en ce que, en tant que solution, on utilise une solution de polyuréthane.

# Fig.1

# Fig.2

# Fig.3

# Fig.4